# EUROPEAN PATENT APPLICATION

(11) **EP 3 593 811 A1**
(43) Date of publication of application: **15.01.2020**
(21) Application number: 19191447.2
(22) Date of filing: 12.08.2013
(51) Int. Cl.: A61K 35/744, A61K 35/745, A61K 35/747, A61P 3/04, A61P 3/08, A61P 3/10, A61P 9/10

(54) **PROBIOTIC COMPOSITIONS AND METHODS FOR THE TREATMENT OF OBESITY AND OBESITY-RELATED CONDITIONS**

(30) Priority: 20.09.2012 US 201261703257 P
(62) Divisional of application: 13839430.9
(71) Applicant: Prothera, Inc., Reno, Nevada 89521 (US)
(72) Inventor: Olmstead, Stephen, Francis, Damascus, Oregon 97089 (US)
(74) Representative: Schüssler, Andrea

(57) **Abstract**

The present invention relates, in general, to combinations of probiotic organisms for the treatment of obesity, diabetes and obesity-related conditions. Also, this invention relates to methods for combining and using probiotic organisms to prevent and treat obesity, diabetes and cardiovascular disease.

## Description

This application claims the priority benefit under 35 U.S.C. section 119 of U.S. Provisional Patent Application No. 61/703,257 entitled "Probiotic Compositions And Methods For The Treatment Of Obesity And Obesity-Related Conditions" filed on September 20, 2012; which is in its entirety herein incorporated by reference.

### FIELD OF THE INVENTION

The present invention relates, in general, to combinations of probiotic organisms for the treatment of obesity, diabetes and obesity-related conditions. Also, this invention relates to methods for combining- and using probiotic organisms to prevent and treat obesity, diabetes and cardiovascular disease.

### BACKGROUND OF THE INVENTION

Obesity, once an uncommon condition, is now pandemic. The World Health Organization currently estimates 1.4 billion adults worldwide are overweight. Of these, an alarming 200 million men and 300 million women are obese. Obesity is associated with a constellation of physiological disorders such as insulin resistance, type 2 diabetes mellitus, hypertension, dyslipidemia, cardiovascular disease, and metabolic syndrome. The medical costs associated with obesity in the U.S. have been estimated at $147 billion per year. Safe and effective interventions are urgently needed to combat the medical problems and costs associated with obesity.

Once simplistically considered a disorder caused by an imbalance of energy intake (caloric consumption) versus energy expenditure (physical activity/exercise), obesity is now viewed as a complex, multifactorial disorder. Among other factors, the increased use of high-fructose corn syrup in the United States mirrors the rapid increase in obesity (Bray et al., 2004). In fact, studies in rats have shown that type II diabetes and hypertension can be induced by feeding a high-fructose diet (Hwang et al., 1987). The digestion, absorption and metabolism of fructose differ from those of glucose. Hepatic metabolism of fructose favors de novo lipogenesis. In addition, unlike glucose, fructose does not stimulate insulin secretion or enhance leptin production. Because insulin and leptin act as key signals in the regulation of food intake and body weight, this suggests that dietary fructose may contribute to increased energy intake and weight gain.

Many previous studies have shown that probiotic bacteria support the growth of beneficial gut bacteria colonies but it also seems that certain beneficial probiotic strains can also alter host metabolism pathways for the better. Microbial organisms produce bioactive substances that influence carbohydrate and lipid metabolism, and modulate both intestinal and systemic inflammatory processes. Thus, there has been increasing interest in identifying nutritional supplements and probiotic foods that are effective for the control of obesity and diabetes (for a review, see Mallappa et al., 2012). In particular, methods are needed to identify probiotic organisms that be combined to produce effective treatments for these serious conditions.

It has long been known that the gut microbiota extracts energy from dietary substances indigestible by the host. Dietary components that escape digestion by endogenous enzymes in the upper gastrointestinal tract become available as substrates in the large intestine. These non-digestible dietary carbohydrates include resistant starch, plant cell wall material, and oligosaccharides. Also, several studies indicate that fructose is not completely absorbed in the small intestine; undigested fructose is transported into the large intestine, where it is fermented by the colonic flora. In addition, several heterofermentative bacteria are capable of converting fructose to mannitol (Wisselink et al., 2002).

Yadav et al. (2007) studied the progression of type II diabetes in rats fed high-fructose diets; they observed that a diet supplemented with *Lactobacillus acidophilus* and *Lactobacillus casei* delayed the onset of glucose intolerance, hyperglycemia, and hyperinsulinemia. Andreasen et al. (2010) reported that a strain of *Lactobacillus acidophilus* preserved insulin sensitivity among volunteers with type II diabetes, whereas insulin sensitivity decreased in the placebo group. Kadooka et al. (2010) observed a slight but statistically significant effect of a strain of *Lactobacillus gasseri* on abdominal adiposity, body weight and other body measures in adults with obese tendencies. However, Arora et al. (2012) found no effect of a single probiotic agent, *Lactobacillus acidophilus* NCDC 13, on weight loss in obese subjects. Also, Murphy et al. (2012) observed no improvement in metabolic profiles in obese mice fed *Lactobacillus salivarius* strain UCC118.

Studies performed to date have focused primarily on single probiotic species. To date, no group has described a systematic method for combining probiotic microorganisms to improve the efficacy of probiotic compositions. However, we have observed that certain mixtures of two or more probiotic microorganisms, if given together, are more effective than individual species. In particular, we have found that a probiotic microorganism that metabolizes carbohydrates via the Embden-Myerhof pathway (EMP) or a phosphoketolase pathway (PKP) can be combined with a probiotic microorganism that metabolizes carbohydrates via a fructose-6-phosphate pathway (F6PPK) to produce synergistic effects. These metabolic pathways are known to those skilled in the art, but the use of complementary metabolic pathways to design effective treatments for obesity has not been described in the prior art.

In sum, there has gone unmet a need for improved methods, compositions, etc. that can prevent weight, gain and ameliorate one or more symptoms and signs associated with obesity. Effective dietary and/or pharmaceutical interventions for these conditions could have a major public health
impact. The present systems and methods, etc., provide these and/or other advantages.

### OBJECTS OF THE INVENTION

It is an object of the present invention to provide mixtures of probiotic microorganisms that are safe and effective for the prevention of weight gain and the treatment of obesity, diabetes, hypertension and cardiovascular disease.

Another object of the invention is to teach methods for combining probiotic microorganisms to create synergistic compositions.

These and other objects of the present invention will become more readily apparent from the description and examples which follow.

### SUMMARY OF THE INVENTION

In one aspect, the compositions, methods, systems, etc., herein are directed to providing probiotic compositions that are capable of maintaining or reducing body weight or body mass index (BMI), preventing or treating obesity and/or obesity-related conditions. The compositions can also be used to prevent excessive weight gain during pregnancy.

The compositions, formulations, methods, etc., provided herein can be used as dietary supplements or as food additives or as pharmaceutical agents or otherwise as desired to achieve these aims. The methods, etc., herein include methods, kits, labels, systems, etc., directed to labeling, marketing and otherwise providing the compositions to health care professionals and/or to consumers for use in this application.

The compositions may be used as dietary supplements, food and beverage additives, and as pharmaceutical agents for reducing the symptoms of obesity, diabetes and/or obesity-related conditions in a human in need thereof.

The inclusion of a first probiotic microorganism that metabolizes carbohydrates via a homofermentative or heterofermentative pathway (EMP or PKP) and a second probiotic microorganism that metabolizes carbohydrates via a fructose-6-phosphate pathway (F6PPK) is essential for this invention. The first microorganism may be a homofermentative or heterofermentative lactic acid bacterium, preferably a species of *Lactobacillus.*
In a further embodiment, the compositions, etc., are provided in capsules or other suitable administration formats, and a single capsule provides a full serving or dose. Generally speaking, a serving is an individual, full quantity of food or drink. Nutritional supplements and the like are typically considered foods, and thus herein the term "serving" is the term used for a full portion of supplement, which can be, for example, 1 capsule, 1/4 teaspoon, or 6 tablets. Dose is a full quantity of medication to be taken at one time. As used herein, both indicate a full portion to be taken by or administered to a recipient at a single time.

In general, probiotic yields are 100-450 billion Colony Forming Units (CFU) per gram. In one example, each serving or dose comprises at least about 1 billion and up to 50 billion Colony Forming Units (CFU) of active microorganisms per 1 capsule serving. For higher serving doses, powders can be used. For example, Ther-Biotic Complete Powder (ProThera, Inc.) has 400 billion CFU per teaspoon.

In a further embodiment, the first microorganism is one or more of *Lactobacillus acidophilus (L. acidophilus), L. brevis, L. bulgaricus, L. casei, L. crispatus, L. curvatus, L. fermentum, L. gasseri, L. helveticus, L. johnsonii, L. paracasei, L. pentosus, L. plantarum, L. reuteri, L. rhamnosus, L. salivarius, L. sakei;* and the second microorganism is one or more *of B. bifidum, B. breve, B. lactis, B. longum,* or *B. infantis.* Alternatively, the second microorganism may be *Leuconostoc mesenteroides* (and subspecies thereof, such as *Leuconostoc pseudomesenteroides* and *Leuconostoc mesenteroides* ssp. *cremoris*)*.*

In one embodiment, the selected species of *Lactobacillus* and *Bifidobacterium* or *Leuconostoc* is combined with one or more further probiotics. The additional probiotic may be any microorganism that has a beneficial effect on obesity and/or obesity-related conditions. Typically, the additional probiotic is one or more of: *Lactobacillus acidophilus, L. brevis, L. bulgaricus, L. casei, L. crispatus, L. curvatus, L. fermentum, L. gasseri, L. helveticus, L. johnsonii, L. paracasei, L. humans paraplantarum, L. pentosus, L. plantarum, L. reuteri, L. rhamnosus, L. salivarius, L. sakei, B. animalis, B. bifidum, B. breve, B. lactis, B. longum, B. infantis, Streptococcus thermophilus, Saccharomyces boulardii,* and *Saccharomyces cereviseae.*

In a further embodiment, the composition can be a dietary supplement which is administered as a dried powder, a tablet, a hydroxypropyl methylcellulose capsule, or a gelatin capsule. Exemplary methods for encapsulation of probiotics can be found, e.g., in US Patent Appl. 2007/0122397.

In a further embodiment, the composition can be provided within a food or beverage suitable for human consumption. For the purpose of this invention, exemplary food and beverage products include a cereal based product, rice cake, soy cake, food bar product, cold formed food bar product, custard, pudding, gelatin, rice milk, soy milk, almond milk, yogurt, kefir, juice, mashed fruit product, candy, candy bar, and applesauce.

In one embodiment, none of the probiotic organisms in the composition have been or are propagated or grown in media containing casein or gluten.

In an additional embodiment, the composition can be a pharmaceutical composition, subject to FDA approval. The pharmaceutical compositions, capsules, etc., herein are contained in a pharmaceutically acceptable container. As a pharmaceutical composition, the product can be marketed and dispensed together with the written description, brochure, information sheet, catalog, or label explaining the product can reduce one or more symptoms of obesity and/or the product is free of casein and gluten. In an additional embodiment the product is marketed together with a written description, brochure, information sheet, catalog, or label explaining that the product is hypoallergenic. The label can be an FDA approved label.

The nutritional and/or pharmaceutical composition that is the subject of the present invention further can comprise at least one prebiotic agent that promotes the growth of probiotic microorganisms in the gastrointestinal tract. The prebiotic agent can comprise at least one of a fructooligosaccharide, galactooligosaccharide, lactulose, beta-glucan, inulin, pectin and resistant starch.

The nutritional and/or pharmaceutical composition further can comprise conjugated linoleic acid (CLA) isomers, containing conjugated double bonds. Conjugated linoleic acid (CLA) is a group of polyunsaturated fatty acids found in beef, lamb, and dairy products that exist as positional and stereoisomers of octadecadienoate (18:2) (Caescu et al., 2004). Various health benefits have been attributed to CLA in experimental animal models including actions to reduce carcinogenesis, atherosclerosis, onset of diabetes, and body fat mass. The most bioactive CLA isomers are cis-9, trans-11, trans-10 and cis-12.

The nutritional and/or pharmaceutical composition further can contain chromium. Chromium, as a nutritional supplement, is used to improve blood sugar control in people with prediabetes, type 1 and type 2 diabetes, and high blood sugar due to taking steroids. Two easily absorbed forms of chromium are chromium polynicotinate or chromium picolinate or chromium histidinate. As an example, the compositions contemplated herein can contain chromium polynicotinate at a dose of approximately 500-1000 µg per capsule or dose.

The obesity-related condition that is the subject of the invention can be one or more of hyperglycemia, insulin resistance, diabetes, hypertriglyceridemia, hypercholesterolemia, atherosclerosis, angina pectoris, arterial occlusion, myocardial infarction and/or stroke.

In addition, the compositions and methods, etc. of the invention can be used to ameliorate or prevent excessive weight gain during pregnancy. The Institute of Medicine recommends a weight gain of 25-35 pounds for women of normal weight, 28-40 pounds for those considered underweight, 15-25 pounds for overweight women and no more than 15 pounds for obese women. A woman with a normal BMI (Body Mass Index) of 20-25 should attempt to follow this weight gain schedule:

| WEEK TOTAL | CUMULATIVE GAIN | EXAMPLE (140 pound woman) |
|---|---|---|
| 15 weeks | 2-5 pounds | Total Weight: 142-145 pounds |
| 20 weeks | 6-11 pounds | Total Weight: 146-151 pounds |
| 25 weeks | 11-17 pounds | Total Weight: 151-157 pounds |
| 30 weeks | 16-23 pounds | Total Weight: 156-163 pounds |
| 35 weeks | 20-28 pounds | Total Weight: 160-168 pounds |
| 40 weeks | 25-35 pounds | Total Weight: 165-175 pounds |

Accordingly, "excessive weight gain" can be defined as weight gain that exceeds the guidelines shown above.

The compositions and methods, etc. can be used to ameliorate or prevent gestational diabetes. Gestational diabetes is defined as "any degree of glucose intolerance with onset or first recognition during pregnancy" (Metzger et al., 1998).

In still other aspects, this application is directed to isolated and purified compositions as described herein for use in the manufacture of a medicament for reducing or maintaining body mass index (BMI) or for inhibiting, preventing, or treating obesity or an obesity-related condition, as well as methods of manufacturing such medicaments, which can comprise combining a pharmaceutically effective amount of the composition and a pharmaceutically acceptable capsule, tablet, powder or liquid.

These and other aspects, features and embodiments are set forth within this application, including the following Detailed Description. Unless expressly stated otherwise, all embodiments, aspects, features, etc., can be mixed and matched, combined and permuted in any desired manner.

### DETAILED DESCRIPTION OF THE INVENTION

The body mass index (BMI) (calculated as weight in kilograms divided by the square of height in meters) is the most commonly accepted measurement for overweight and/or obesity. In adults, a BMI exceeding 25 is considered overweight, while obesity is defined as a BMI of 30 or more, with a BMI of 35 or more considered as serious co-morbidity and a BMI of 40 or more considered morbid obesity. For the purposes of this invention, "obesity" shall mean a BMI of 30 or more.

One out of every five overweight people is affected by the "metabolic syndrome". Metabolic syndrome is one of the fastest growing obesity-related health concerns in the United States and is characterized by a cluster of health problems including obesity, hypertension, abnormal lipid levels, and high blood sugar. According to the Centers for Disease Control and Prevention (CDC), the metabolic syndrome affects almost one quarter (22 percent) of the American population - an estimated 47 million people. The assemblage of problems characterized as comprising the metabolic syndrome can increase a patient's risk for developing more serious health problems, such as diabetes, heart disease, and stroke.

Overweight and obese people have an increased incidence of heart disease, and thus fall victim to heart attack, congestive heart failure, sudden cardiac death, angina, and abnormal heart rhythm more often than those that maintain a healthy body mass index. Obesity often increases the risk of heart disease because of its negative effect on blood lipid levels, which increase in obese patients and then, in turn, increase triglyceride levels and decrease high-density lipoprotein - which is also known as HDL. People with an excessive amount of body fat have higher levels of triglycerides and low-density lipoprotein - which is also known as LDL or "bad cholesterol" - as well as lower levels of HDL cholesterol in the blood. This combination creates optimal conditions for developing atherosclerotic heart disease.

Being overweight or obese increases the risk of developing high blood pressure. Hypertension, or high blood pressure, greatly raises the risk of heart attack, stroke, and kidney failure. In fact, blood pressure rises as body weight increases. Losing even 10 pounds can lower blood pressure-and losing weight has the biggest effect on those who are overweight and already have hypertension.

Obesity is associated with the development of diabetes. More than 80 percent of people with type 2 diabetes, the most common form of the disease, are obese or overweight. Type 2 diabetes develops when either there is impaired insulin production by the pancreas in the setting of insulin resistance in the tissues and organs in the body. As obesity diminishes insulin's ability to control blood sugar (glucose), there is an increased risk of developing diabetes because the body begins overproducing insulin to regulate blood sugar levels. Over time, the body is no longer able to keep blood sugar levels in the normal range. Eventually the inability to achieve healthy blood sugar balance results in the development of type 2 diabetes. Furthermore, obesity complicates the management and treatment of type 2 diabetes by increasing insulin resistance and glucose intolerance, which makes drug treatment for the disease less effective. In many cases, a reduction of body weight to a normal range normalizes blood glucose and restores insulin sensitivity.

Childhood obesity is also a major public health problem, particularly in Western countries. Children 2-18 years of age are considered obese if the BMI is greater than the 95th percentile. Despite policies targeted at reducing its prevalence, childhood obesity has more than doubled in children and tripled in adolescents in the past 30 years. As with adults, obesity in childhood causes hypertension, dyslipidaemia, chronic inflammation, increased blood clotting tendency, endothelial dysfunction, and hyperinsulinemia. This clustering of cardiovascular disease risk factors has been identified in children as young as 5 years of age. Thus there is an urgent need for safe effective interventions, including nutritional interventions, to combat the epidemic of obesity in children as well as in adults.

The present compositions, medicaments, therapeutics, systems, methods, etc., are directed to the prevention, inhibition and treatment of obesity and obesity- related conditions. Said obesity-related conditions are selected from the group consisting of insulin resistance, hyperglycemia, diabetes, hypertriglyceridemia, atherosclerosis, angina pectoris, myocardial infarction and/or stroke.

### Probiotic Compositions

"Probiotics" within the context of the present invention is used in accord with its usual meaning, for example as selected, viable microbial dietary supplements that, when introduced in sufficient quantities, beneficially affect the human organism via their effects in the gastrointestinal tract (Holzapfel et al., 2001; Holzapfel & Schillinger, 2002). The FAO/WHO has adopted the definition of probiotics as "Live microorganisms which when administered in adequate amounts confer a health benefit on the host" (FAO/WHO guidelines, 2002). These beneficial bacteria may be found for example in milk or in milk processing factories, living or decaying plants, and also in the intestines of man and animals.

Currently, the best-studied probiotics are the lactic acid bacteria, particularly *Lactobacillus* spp. and *Bifidobacterium* spp. *Lactobacillus* is a genus of Gram-positive facultative anaerobic bacteria. The genus *Lactobacillus* currently comprises over 100 species and encompasses a wide variety of organisms. They are common and usually benign. In humans they are present in the vagina and the gastrointestinal tract, where they are symbiotic and make up a small portion of the gut flora (Tannock, 1999). Lactobacilli that have been used in humans include *L. acidophilus, L. salivarius, L. johnsonii, L. casei, L. lactis, L. reuteri, L. plantarum, L. rhamnosus, L. brevis, L. gasseri,* and other species and subspecies. The use of *Lactobacillus* species in humans has been extensively reviewed in the scientific literature, including the references provided herein. These ingredients are readily available from commercial suppliers, including Danisco-Dupont (US); Chr. Hansen (Denmark); Institut Rosell Lallemand (Montreal, Canada); and others. Exemplary species and strains of *Lactobacillus* for the present invention include the following well-known strains: *L. acidophilus* NCFM, *L. acidophilus* La-14, *L. bulgaricus* Lb-64, *L. brevis* Lbr-35, *L. casei* Lc-11, *L. lactis* LI-23, *L. plantarum* Lp-115, *L. paracasei* Lpc-37, *L. rhamnosus* Lr-32 and *L. salivarius* Ls-33, which are well known to those skilled in the art.

*Bifidobacterium* is a genus of Gram-positive anaerobic bacteria, currently comprised of 31 characterized species, 11 of which have been detected in human feces (Tannock, 1999). Bifidobacteria are Gram-positive, irregular or branched rod-shaped bacteria that are commonly found in the intestines of humans and most animals and insects. Probiotic *Bifidobacterium* strains that are useful for the present invention include but are not limited to the following strains which are well known to those skilled in the art: *B. breve* Bb-03, *B. lactis* Bi-07 and Bi-04, *B. longum* Bi-05.

*Leuconostoc* is a genus of Gram-positive bacteria, placed within the family of Leuconostocaceae. All species within this genus are heterofermentative. *Leuconostoc,* along with other lactic acid bacteria such as *Pediococcus* and *Lactobacillus,* is responsible for the fermentation of cabbage making sauerkraut. For the purposes of the present invention, one exemplary strain of *Leuconostoc* is *L. mesenteroides* ATCC 13146.

### Carbohydrate metabolism in lactic acid bacteria

Lactic acid bacteria (LAB) are capable of generating energy by homo- or heterofermentative metabolism of sugars. During anaerobic growth of obligately homofermentative LAB in the presence of excess substrate, energy sources like glucose are converted into pyruvate via the Embden-MeyerhofF-Parnas pathway, and the pyruvate is further metabolized to lactate (see Fig 1). Homofermentative LAB include most species of enterococci, lactococci, pediococci, streptococci, tetragenococci, and vagococci.

Early work demonstrated that fructose 1,6 bisphosphate aldolase (EC 4.1.2.13) and isomerase enzymes were absent in heterofermentative organisms, suggesting that the pathway does not follow the usual Embden-Meyerhof pattern of glycolysis (DeMoss et al., 1951). As more research was conducted, it was realized that these organisms utilize a different pathway, named the phosphoketolase pathway (PKP; EC 4.1.2.9), which produces equimolar amounts of CO₂, lactate, and acetate-ethanol (Fig 2).

Heterofermentative LAB can be divided into obligately heterofermentative species, in which both hexoses and pentoses are fermented via the PKP, and facultatively heterofermentative organisms, which degrade hexoses via the Embden-Meyerhoff-Parnas pathway and pentoses via the PKP. Many of the enzymes used in the latter pathway are shared with the pentose phosphate pathway.

Xylulose 5-phosphate phosphoketolase (XPK; EC 4.1.2.9) is the central enzyme of the PKP of heterofermentative and facultative homofermentative lactic acid bacteria. XPK prefers xylulose 5-phosphate to fructose 6-phosphate. In the presence of inorganic phospate this enzyme converts xylulose 5-phosphate (X5P) into glyceraldehyde 3-phosphate and acetylphosphate. Some taxa known to possess the PKP pathway include *Lactobacillus brevis, Lactobacillus buchneri, Lactobacillus casei, Lactobacillus ferrnentum, Lactobacillus reuteri, Leuconostoc lactis, Leuconostoc mesenteroides, Leuconostoc mesenteroides* ssp. *cremoris,* and some species of *Weissella.*

Lactobacilli can be grouped into one of these categories:
1) Obligately homofermentative (Group I) including: *L. acidophilus, L. delbrueckii, L. helveticus, L. salivarius*
2) Facultatively heterofermentative (Group II) including: *L. casei, L. curvatus, L. plantarum, L. sakei*
3) Obligately heterofermentative (Group III) including: *L. brevis, L. buchneri, L. fermentum, L. reuteri*

Bifidobacteria are considered key commensals in human-microbe interactions and they contribute to the degradation of undigested polysaccharides in the human colon (Suzuki et al., 2010). Bifidobacteria utilize a unique pathway of hexose catabolism which produces primarily acetate and lactate (de Vries and Stouthamer, 1967). This fermentation pathway, which is known as the "Bifid shunt" or the "fructose-6-phosphate pathway" yields 3 mols of acetate and 2 mols of lactate for 2 mols of glucose, with production of 5 mols of ATP. The key enzyme in the pathway is xylulose-5-phosphate phosphoketolase/fructose-6-phosphate phosphoketolase (Xfp; EC 4.1.2.22), which catalyzes two important steps: splitting D-fructose 6-phosphate into D-erythrose 4-phosphate and acetylphosphate, and splitting D-xylulose 5-phosphate into D-glyceraldehyde 3-phosphate and acetylphosphate. This enzyme has often been used as a tool in the identification of bifidobacteria. More recently, such dual substrate-specificity enzymes have been found in other organisms including *Leuconostoc mesenteroides* and *Lactobacillus paraplantarum* (Lee et al. 2005; Jeong et al., 2007).

In addition, *Bifidobacterium longum,* which metabolizes intracellular fructose via the fructose-6-P phosphoketolase pathway, contains a fructokinase (Frk; EC 2.7.1.4) (Caescu et al. 2004). Fructokinases have also been found in *Leuconostoc mesenteroides, Leuconostoc pseudomesenteroides, Lactobacillus plantarum,* and *Lactococcus lactis.* The presence of fructokinase enables these organisms to grow using fructose as a unique carbon source. Furthermore, a number of heterofermentative lactic acid bacteria (LAB), yeasts, and filamentous fungi also are known to convert fructose into mannitol in significant quantities, including *Leuconostoc mesenteroides.* The reduction of fructose to mannitol in heterofermentative lactic acid bacteria is catalyzed by an NADH-linked mannitol dehydrogenase (EC 1.1.1.67) (Wisselink et al., 2002; Saha & Racine, 2011).

In clinical practice we have discovered that certain combinations of lactic acid bacteria, if given together, are far more effective than if either species is given alone. The basis of the present invention is the observation that compositions comprising two or more probiotic organisms, with distinct pathways of carbohydrate metabolism, produce synergistic results with respect to weight loss and diabetes. Specifically, we have discovered that combination products containing at least one species of *Lactobacillus* and at least one species of *Bifidobacterium* or *Leuconostoc* are effective in the treatment of obesity, diabetes, and obesity-related conditions.

No one has previously recognized that distinct carbohydrate metabolic pathways can be used to design synergistic compositions for the treatment of obesity. Without being bound by theory, we propose that the beneficial effects of our compositions may result from the efficient metabolism of carbohydrates by the combined action of these particular species; from the combination of short-chain fatty acids or other metabolites produced by the complementary species; or by another mechanism.

**Table 1. Compositions for the treatment of obesity and diabetes**

| **Composition** | ***Lactobacillus* sp. (per capsule or serving**) | ***Bifidobacterium* sp. (per capsule or serving)** | **Other sp. (per capsule or serving)** |
|---|---|---|---|
| Composition 1 | *L. acidophilus, L. rhamnosus, L. casei, L. plantarum, L. salivarius* (2.5+ billion CFU of each) | *B. bifidum, B. longum, B. lactis. B. breve (1.0*+ *billion CFU of each)* | *Streptococcus thermophilus (1.0*+ *billion CFU)* |
| Composition 2 | *L. rhamnosus, L. casei, L. salivarius, L. paracasei* (2.0+ billion CFU of each) | *B. bifidum, B. longum, B. breve, B. infantis* (2.0+ billion CFU of each) | |
| Composition 3 | *L. acidophilus, L. rhamnosus,* L*.* brevis (1.0+ billion CFU of each) | *B. bifidum, B. lactis,* B. infantis (1.0+ billion CFU of each) | |
| Composition 4 | *L. acidophilus, L. rhamnosus* (1.5+ billion CFU of each) | *B. bifidum, B. lactis* (1.5+ billion CFU of each) | *Leuconostoc mesenteroides* (1.0 + billion CFU) |
| Composition 5 | *L. acidophilus,* 2.5+ billion CFU | *B. bifidum,* 2.5+ billion CFU | - |
| Composition 6 | *L. acidophilus, L. rhamnosus,* 1.25+ billion CFU of each | *B. bifidum, B. lactis,* 1.25+ billion CFU of each | - |
| Composition 7 | *L. acidophilus* (6.3+ billion CFU), L*.* rhamnosus (9.4+ billion CFU), L*.* bulgaricus (1.5+ billion CFU) | *B. bifidum,* 6.3+ billion CFU | *Streptococcus thermophilus* 1.5+ billion CFU |
| Composition 8 | *L. rhamnosus, L. casei, L. salivarius, L. paracasei* (17+ billion CFU of combined *Lactobacillus* species) | *B. bifidum, B. longum, B. breve, B. infantis* (8+ billion CFU of combined *Bifidobacterium* species) | - |
| Composition 9 | *L. acidophilus, L. rhamnosus, L. paracasei* (100+ billion CFU of combined *Lactobacillus* and *Bifidobacterium* species) | *B. bifidum, B. longum, B. lactis* | - |
| Composition 10 | *L. acidophilus* (1.85+ billion CFU), *L. rhamnosus, L. bulgaricus, L. brevis, L. casei, L. salivarius, L. plantarum* (4.9+ billion CFU combined with *S. thermophilus*) | B. bifidum (0.70+ billion CFU), B. lactis | Streptococcus thermophilus |
| Composition 11 | *L. rhamnosus* (2.5+ billion CFU) | *B. bifidum, B. breve* (1.25+ billion CFU) | *Saccharomyces boulardii* (5.0+ billion CFU) |

### EXAMPLE 1

A 47 year-old man presents for evaluation of obesity. He has been gaining weight since his early 40s. He weighs 280 pounds and is 5 foot 11 inches tall with a body mass index (BMI) of 39. His blood pressure is 140/90. Laboratory testing is remarkable for a fasting blood glucose of 136 mg/dL and triglycerides of 220 mg/dL. A diet is recommended consisting of high protein, reduced refined carbohydrates, and 2200 calories per day. A 4-day-per-week minimum program of aerobic exercise is prescribed. The patient is given a multispecies *Bifidobacterium*/ *Lactobacillus* probiotic formula, (Composition 1; see Table 1) in the amount of two capsules per day to be taken with meals. When the patient is seen in follow up after 3 months, his weight is 232 with a BMI of 32.4. His blood pressure is now 130/84 and his glucose and triglycerides are normal. He is advised to continue his diet, exercise and probiotics. When he is seen again in 6 months, he weighs 189 pounds with a BMI of 26.8.

### EXAMPLE 2

A 62-year old woman presents for evaluation of obesity. She weighs 191 pounds and is 5 foot 6 inches tall with a body mass index (BMI) of 31. She reports consuming a diet consisting of approximately 2200 calories per day and walking for 30 minutes three to four days per week. For the past three months she has been consuming 3 billion CFU/day of a commercial *Lactobacillus acidophilus* supplement; however, she has been unable to lose weight. The patient is given a multispecies probiotic formula containing *Bifidobacterium* and *Leuconostoc* in addition to *Lactobacillus* (Composition 4; see Table 1) in the amount of one capsule per day to be taken with a meal. When the patient is seen at followup in 3 months her weight is 175 pounds with a BMI of 28.

### EXAMPLE 3

A 30-year-old pregnant woman presents for evaluation of excessive weight gain and gestational diabetes in her 28^{th} week of pregnancy. She is 5'4" tall and weighs 163 pounds, having gained 40 pounds during her pregnancy. An oral glucose tolerance test reveals a plasma glucose level of 12 mmol/L when measured 2 hours after the challenge, suggesting overt diabetes. The patient is advised to avoid high-sugar foods, like sweets and desserts; to increase her daily intake of dietary fiber and protein; and to incorporate at least 40 minutes per day of gentle exercise in her routine. In addition, the patient is given a *Lactobacillus*/*Bifidobacterium* probiotic formula, consisting of *L. acidophilus, L. rhamnosus, B. bifidum,* and *B. lactis* (Composition 3; see table 1) and advised to take one capsule per day with a meal. When the patient is seen at followup in 2 weeks her weight has stabilized at 163 pounds and her plasma glucose level has decreased to 10.5 mmol/L when measured 2 hours after a glucose challenge.

### EXAMPLE 4

A 10-year-old female child is seen at a pediatric clinic for evaluation of obesity. At birth, she weighed 9 pounds and was 20 inches in length. Even in infancy the child had risk factors for obesity as a result of a family history of the disease. Also, the patient's mother had gestational diabetes, which can predispose a child to overweight/obesity. Children 2-18 years of age are considered obese if the BMI is greater than the 95th percentile. During early childhood, the patient's weight was maintained in the 90^{th} to 95^{th} percentile. However, she continued to grow, and by her 10^{th} birthday she was considered overweight with a height of 50 inches, weight of 85 pounds, and BMI of 24 which is in the 96^{th} percentile according to CDC guidelines (Centers for Disease Control). A dietitian advises the mother to modify the child's diet by limiting snack foods and providing fresh fruit for dessert instead of cookies. The dietitian also advises a probiotic supplement, (Composition 2; see Table 1), which contains a mixture of *Lactobacillus* and *Bifidobacterium,* in the amount of one capsule per day to be consumed with a meal. When seen at follow-up 6 months later the patient's height is 52 inches and her weight is 74 pounds, which represents a weight loss of 11 pounds. The patient's BMI is now 19.2, placing the BMI-for-age at the 76th percentile. The patient's mother is advised to continue the diet and probiotic regimen and to encourage the child to participate in a sports, dance or an exercise program.

The invention is further described by the embodiments of the following paragraphs:
1. A composition for preventing or ameliorating obesity or an obesity-related condition; said composition containing, as an active ingredient, at least two species of probiotic microorganisms, wherein the first species is selected from the group comprising *Lactobacillus* and the second species is selected from the group comprising *Bifidobacterium or Leuconostoc,* in a capsule, tablet, dry powder, food or beverage.
2. A composition for preventing or ameliorating obesity or an obesity-related condition; said composition containing, as an active ingredient, at least two probiotic microorganisms with different pathways of carbohydrate metabolism, wherein at least one probiotic microorganism contains a homofermentative or heterofermentative pathway of carbohydrate metabolism, and at least one probiotic microorganism contains a fructose-6-phosphate phosphoketolase.
3. A nutritional or food supplement to assist in the maintenance or reduction of (a) body weight and/or (b) blood glucose; said composition containing, as active ingredients, at least two species of probiotic microorganisms, wherein the first species is selected from the group comprising Lactobacillus and the second species is selected from the group comprising Bifidobacterium or Leuconostoc, in a capsule, tablet, dry powder, food or beverage.
4. A nutritional or food supplement to assist in the maintenance or reduction of (a) body weight and/or (b) blood glucose; said composition containing, as active ingredients, at least two probiotic microorganisms with different pathways of carbohydrate metabolism, wherein at least one probiotic microorganism contains a homofermentative or heterofermentative pathway of carbohydrate metabolism, and at least one probiotic microorganism contains a fructose-6-phosphate phosphoketolase.
5. A composition for preventing or ameliorating obesity or an obesity-related condition; said composition containing, per capsule or serving, at least 2.5 billion CFU each *of L. acidophilus, L. rhamnosus, L. casei, L. plantarum, and L. salivarius; at least 1 billion CFU each of B. bifidum, B. longum, B. lactis,* and *B. breve;* and at least 1 billion CFU of *Streptococcus thermophilus.*
6. A composition for preventing or ameliorating obesity or an obesity-related condition; said composition containing, per capsule or serving, at least 2 billion CFU each of *L. rhamnosus, L. casei, L. salivarius, L. paracasei, B. bifidum, B. longum, B. breve,* and *B. infantis.*
7. A composition for preventing or ameliorating obesity or an obesity-related condition; said composition containing, per capsule or serving, at least 1 billion CFU each of L. *acidophilus, L. rhamnosus, L. brevis, B. bifidum, B. lactis,* and *B. infantis.*
8. A composition for preventing or ameliorating obesity or an obesity-related condition; said composition containing, per capsule or serving, at least 1.5 billion each *of L. acidophilus, L. rhamnosus, B. bifidum* and *B. lactis;* and at least 1 billion CFU of *Leuconostoc mesenteroides.*
9. The composition according to paragraph 1, wherein the obesity-related condition is selected from the group comprising hyperglycemia, insulin resistance, diabetes, hypertriglyceridemia, hypercholesterolemia, atherosclerosis, angina pectoris, myocardial infarction and/or stroke.
10. The composition according to paragraph 1, comprising three or more probiotic microorganisms selected from the group consisting of *Lactobacillus, Bifidobacterium, Streptococcus, Saccharomyces,* and *Leuconostoc.*
11. The composition according to paragraph 1, wherein the first probiotic microorganism is selected from the group comprising *Lactobacillus rhamnosus, Lactobacillus acidophilus, Lactobacillus casei, Lactobacillus plantarum, Lactobacillus bulgaricus, Lactobacillus paracasei, Lactobacillus reuteri, Lactobacillus salivarius, Lactobacillus gasseri, Lactobacillus brevis,* and *Lactobacillus fermentum.*
12. The composition according to paragraph 1, wherein the second probiotic microorganism is selected from the group comprising *Bifidobacterium bifidum, Bifidobacterium longum, Bifidobacterium lactis, Bifidobacterium breve, Bifidobacterium infantis,* and *Leuconostoc mesenteroides.*
13. The composition according to paragraph 1, wherein the composition further comprises at least one of *Streptococcus thermophilus, Saccharomyces boulardii* or *Saccharomyces cerevisiae.*
14. The composition according to paragraph 1, wherein the composition comprises *Lactobacillus acidophilus* and *Bifidobacterium bifidum.*
15. The composition according to paragraph 1, wherein the composition comprises *Lactobacillus acidophilus, Lactobacillus rhamnosus, Bifidobacterium bifidum,* and *Bifidobacterium lactis.*
16. The composition according to paragraph 1, wherein the composition comprises *Lactobacillus rhamnosus, Bifidobacterium bifidum, Lactobacillus acidophilus, Streptococcus thermophilus,* and *Lactobacillus bulgaricus.*
17. The composition according to paragraph 1, wherein the composition comprises *Lactobacillus rhamnosus, Lactobacillus casei, Lactobacillus salivarius, Lactobacillus paracasei, Bifidobacterium bifidum, Bifidobacterium longum, Bifidobacterium breve,* and *Bifidobacterium infantis.*
*18.* The composition according to paragraph 1, wherein the composition comprises *Bifidobacterium longum, Lactobacillus acidophilus, Lactobacillus rhamnosus, Bifidobacterium bifidum, Bifidobacterium lactis,* and *Lactobacillus paracasei.*
19. The composition according to paragraph 1, wherein the composition comprises *Lactobacillus rhamnosus, Lactobacillus bulgaricus, Lactobacillus brevis, Lactobacillus casei, Lactobacillus salivarius, Lactobacillus plantarum, Bifidobacterium lactis,* and *Streptococcus thermophilus.*
20. The composition according to paragraph 1, wherein the composition comprises *Saccharomyces boulardii, Lactobacillus rhamnosus, Bifidobacterium bifidum,* and *Bifidobacterium breve.*
21. The composition according to paragraph 1, wherein the composition further comprises a conjugated linoleic acid molecule.
22. The composition according to paragraph 1, wherein the composition further comprises a chromium salt.
23. The composition according to paragraph 1, wherein the chromium salt is selected from the group consisting of chromium picolinate, chromium histidinate, chromium nicotinate and mixtures thereof.
24. The composition according to paragraph 23, wherein the chromium salt is administered in the range of 50-1000 µgs.
25. The composition according to paragraph 1, wherein the probiotic microorganisms are provided as a capsule, tablet, powder, liquid, beverage, or food product.
26. The composition according to paragraph 1, wherein the microorganisms comprise at least 1 billion colony forming units (CFU) per dose, capsule, tablet, or serving.
27. A method to treat or prevent obesity or an obesity-related condition, to support weight loss, and/or to reduce the body mass index in an individual in need thereof, comprising the step of administering to said individual a composition according to paragraph 1.
28. A method to treat or prevent diabetes and/or to normalize blood glucose in an individual in need thereof, comprising the step of administering to said individual a composition according to paragraph 1.
29. The method of paragraph 28, further including chromium picolinate.
30. A method to treat or prevent one or more signs or symptoms of cardiovascular disease, comprising the step of administering to an individual a composition according to paragraph 1.
31. A method according to paragraph 1, wherein the signs or symptoms of cardiovascular disease are selected from the group comprising hypertriglyceridemia, hypercholesterolemia, atherosclerosis, angina pectoris, arterial occlusion, myocardial infarction and/or stroke.
32. A method of combining probiotic microorganisms for use in the treatment of obesity and obesity-related conditions, said method comprising (1) identifying at least one probiotic microorganism that contains an Embden Myerhof pathway or a phosphoketolase pathway of carbohydrate metabolism; (2) identifying at least one probiotic microorganism that contains a fructose-6-phosphate phosphoketolase pathway of carbohydrate metabolism; (3) combining the at least first and second microorganisms in a capsule, tablet, powder, food or beverage for administration to humans.

### REFERENCES

**US patent documents**

| **US Patent** | **Date** | **Inventor** |
|---|---|---|
| US 6,641,808 | November, 2003 | Bojrab |
| US 6,942,857 | September 2005 | Song, et al. |

**US Patent Applications**

| **Applications** | **Publication Date** | **Inventor** |
|---|---|---|
| 2011/0123501 | May 26, 2011 | Chou, et al. |
| 2010/0111915 | May 6, 2010 | Isolauri, et al. |
| 2005/0112112 | May 26, 2005 | Park, et al. |
| 2012/0121753 | May 17, 2012 | Kim, et al. |
| 2010/0061967 | March 11, 2010 | Rautonen |
| 2012/0058094 | March 8, 2012 | Blaser, et al. |

### Other references

Arora T, Anastasovska J, Gibson G, Tuohy K, Sharma RK, Bell J, Frost G. Effect of Lactobacillus acidophilus NCDC 13 supplementation on the progression of obesity in diet-induced obese mice. Brit J Nutr 2012;31:1-8.
Andreasen AS, Larsen N, Skovsgaard TP,Berg RMG, Møller K, Svendsen KD, Jakobsen M and Pedersen BK. Effects of Lactobacillus acidophilus NCFM on insulin sensitivity and the systemic inflammatory response in human subjects. Brit J Nutr 2010;104:1831-1838.
Belury MA. Dietary conjugated linoleic acid in health: physiological effects and mechanisms of action. Annu Rev Nutr 2002;22:505-31.
Bray GA, Nielsen SJ, and Popkin BM. Consumption of high-fructose corn syrup in beverages may play a role in the epidemic of obesity. Am J Clin Nutr 2004;79:537-43.
Caescu CI, Olivier Vidal, Krzewinski F, Artenie V, and Bouquelet S. Bifidobacterium longum Requires a Fructokinase (Frk; ATP:d-Fructose 6-Phosphotransferase, EC 2.7.1.4) for Fructose Catabolism. J Bacteriol 2004;186(19):6515-6525.
DeMoss, RD, Bard, RC, Gunsalus, IC (1951). The mechanism of the heterolactic fermentation; a new route of ethanol formation. J Bacteriol 62(4):499-511.
De Vries W, Stouthamer AH. Pathway of glucose fermentation in relation to the taxonomy of bifidobacteria. J Bacteriol 1967;93:574-576.
FAO/WHO (2002) Guidelines for the evaluation of probiotics in food. London, Ontario, Canada, Apr. 30 and May 1, 2002.
Holzapfel WH, et al. Taxonomy and important features of probiotic microorganisms in food and nutrition. Am J Clin Nutr 2001;73(2 Suppl):365S-73S.
Holzapfel W H, Schillinger U. Introduction to pre and probiotics. Food Res Int 2002;35:109-116.
Hwang IS, Ho H, Hoffman BB, Reaven GM. Fructose-induced insulin resistance and hypertension in rats. Hypertension 1987; 10: 512-516.
Kadooka Y, Sato M, Imaizumi K, Ogawa A, Ikuyama K, Akai Y, Okano M, Kagoshima M, Tsuchida T. Regulation of abdominal adiposity by probiotics (Lactobacillus gasseri SBT2055) in adults with obese tendencies in a randomized controlled trial. Eur J Clin Nutr 2010;64(6):636-43.
Lee, JM, Jeong, D-W; Kim, OKMJ; Lee,J-H, Angres, I. et al. Cloning and characterization of the gene encoding phosphoketolase in Leuconostoc mesenteroides isolated from kimchi. Biotechnol Lett 2005;27(12):853-858.
Jeong DW, Lee JM, Lee HJ. Cloning and characterization of a gene encoding phosphoketolase in a Lactobacillus paraplantarum isolated from Kimchi. J Microbiol Biotechnol 2007;17(5):822-9.
Mallappa RH, Rokana N, Duary RK, Panwar H, Batish VK, Grover S. Management of metabolic syndrome through probiotic and prebiotic interventions. Indian J EndocrMetab 2012;16:20-27
Meile L, LM, Geissmann TA, Herensperger M, Teuber M. Characterization of the d-Xylulose 5-Phosphate/d-Fructose 6-Phosphate Phosphoketolase Gene (xfp) from Bifidobacteriumlactis. J Bacteriol 2001;183(9):2929-2936.
Metzger, B. E.; Coustan, D. R. (1998). "Summary and recommendations of the Fourth International Workshop-Conference on gestational diabetes mellitus. The Organizing Committee". Diabetes Care 21 Suppl 2: B161-B167.
Murphy EF, Cotter PD, Hogan A, O'Sullivan O, Joyce A, Fouhy F, Clarke SF, Marques TM, OToole PW, Stanton C, Quigley EM, Daly C, Ross PR, O'Doherty RM, Shanahan F. Divergent metabolic outcomes arising from targeted manipulation of the gut microbiota in diet-induced obesity. Gut 2012;Aug 9[Epub ahead of print]
Parvez S, Malik KA, Ah Kang S, Kim HY. Probiotics and their fermented food products are beneficial for health. J Appl Microbiol 2006;100:1171-85.
Sanders ME. Overview of functional foods: emphasis on probiotic bacteria. Int Dairy J 1998;8:341-347
Tannock GW. Identification of lactobacilli and bifidobacteria. Current Issues Molec Biol 1999;1:53-64.
Vaughan EE, Mollet B, de Vos WM. Functionality of probiotics and intestinal lactobacilli: light in the intestinal tract tunnel. Curr Opin Biotechnol 1999;10:505-510.
Saha BC, Racine FM. Biotechnological production of mannitol and its applications. Appl Microbiol Biotechnol. 2011;89(4):879-91.
Suzuki R, Katayama T, Kim B-J Wakagi T, Shoun H, Ashida H, Yamamoto K and Fushinobu S. Crystal structures of phosphoketolase: thiamine diphosphate-dependent dehydration mechanism. J. Biol. Chem 2010;285:34279-34287.
Wisselink HW, Weusthuis RA, Eggink G, Hugenholtz J, Grobben GJ. Mannitol production by lactic acid bacteria: a review. Int1 Dairy J 2002;12:151-161
Yadav H, Jain S, Sinha PR. Antidiabetic effect of probiotic dahi containing Lactobacillus acidophilus and Lactobacillus casei in high fructose fed rats. Nutrition 2007; 23(1):62-68.
Ziemer CJ, Gibson GR. An overview of probiotics, prebiotics and synbiotics in the functional food concept: perspectives and future strategies. Int Dairy J 1998;8:473-479
Zubillaga M, Weil R, Postaire E, Goldman C, Caro R, Boccio J. Effect of probiotics and functional foods and their use in different diseases. Nutr Res 2001; 21:569-579

## Claims

1. A composition comprising, as active ingredients, *Lactobacillus rhamnosus* and *Bifidobacterium bifidum,* in a capsule, tablet, dry powder, food or beverage, for use in a method for preventing or ameliorating obesity or an obesity-related condition.

2. The composition for use according to claim 1, wherein the composition further comprises *Bifidobacterium infantis.*

3. The composition for use according to claim 2, wherein the composition further comprises *Lactobacillus casei, Lactobacillus salivarius, Lactobacillus paracasei, Bifidobacterium longum* and *Bifidobacterium breve.*

4. The composition for the use according to claim 2 or 3, wherein the composition consists of *Lactobacillus rhamnosus, Lactobacillus casei, Lactobacillus salivarius, Lactobacillus paracasei, Bifidobacterium bifidum, Bifidobacterium infantis, Bifidobacterium longum* and *Bifidobacterium breve.*

5. The composition for use according to claim 3 or 4, wherein the composition contains at least about 2 billion colony forming units (CFU) of each *Lactobacillus* species and of each *Bifidobacterium* species per capsule, tablet, dry powder or serving.

6. The composition for use according to claim 2, wherein the composition further comprises *Lactobacillus acidophilus, Lactobacillus brevis* and *Bifidobacterium lactis.*

7. The composition for use according to claim 2 or 6, wherein the composition consists of *Lactobacillus rhamnosus, Lactobacillus acidophilus, Lactobacillus brevis, Bifidobacterium bifidum, Bifidobacterium infantis* and *Bifidobacterium lactis.*

8. The composition for use according to claim 6 or 7, wherein the composition contains at least about 1 billion colony forming units (CFU) of each *Lactobacillus* species and of each *Bifidobacterium* species per capsule, tablet, dry powder or serving.

9. The composition for use according to any one of claims 6 to 8, wherein the obesity-related condition is gestational diabetes.

10. A composition comprising or consisting of, as active ingredients, *Lactobacillus rhamnosus, Lactobacillus acidophilus, Lactobacillus brevis, Bifidobacterium bifidum, Bifidobacterium infantis* and *Bifidobacterium lactis,* in a capsule, tablet, dry powder, food or beverage, for use in a method for preventing or ameliorating excessive weight gain during pregnancy.

11. The composition for use according to claim 10, wherein the composition contains at least about 1 billion colony forming units (CFU) of each *Lactobacillus* species and of each *Bifidobacterium* species per capsule, tablet, dry powder or serving.
